**Europäisches Patentamt**

**European Patent Office**

**Office européen des brevets**

(19)

(11) Numéro de publication : **0 536 013 B1**

(12)
# FASCICULE DE BREVET EUROPEEN

(45) Date de publication du fascicule du brevet :
**17.08.94 Bulletin 94/33**

(51) Int. Cl.⁵ : **B01J 27/199,** C07C 51/25,
C07C 57/04

(21) Numéro de dépôt : **92402377.3**

(22) Date de dépôt : **01.09.92**

(54) **Nouveau système catalytique et son application à l'oxydéshydrogénation d'acides carboxyliques saturés et l'oxydation d'aldéhydes en acides.**

(30) Priorité : **03.09.91 FR 9110868**

(43) Date de publication de la demande :
**07.04.93 Bulletin 93/14**

(45) Mention de la délivrance du brevet :
**17.08.94 Bulletin 94/33**

(84) Etats contractants désignés :
**AT BE CH DE DK ES FR GB GR IE IT LI LU MC
NL PT SE**

(56) Documents cités :
**EP-A- 0 255 639
EP-A- 0 284 947
FR-A- 2 407 022
US-A- 4 550 212**

(73) Titulaire : **ELF ATOCHEM S.A.
4 & 8, Cours Michelet
La Défense 10
F-92800 Puteaux (FR)**

(72) Inventeur : **Cadot, Emmanuel
75, avenue Paul Vaillant Couturier
F-94250 Gentilly (FR)**
Inventeur : **Daubrege, Franck
102, rue d'Aubervilliers
F-75019 Paris (FR)**
Inventeur : **Herve, Gilbert
Route de Maincourt
F-78320 Levis Saint Nom (FR)**
Inventeur : **Teze, André
3, rue Eudoxie
F-78700 Conflans St Honorine (FR)**

(74) Mandataire : **Chaillot, Geneviève
Cabinet CHAILLOT,
B.P. No. 74
F-92703 Colombes Cédex (FR)**

EP 0 536 013 B1

Jouve, 18, rue Saint-Denis, 75001 PARIS

## Description

La présente invention porte sur un système catalytique à base de molybdène, de phosphore, de vanadium et d'oxygène, elle a plus particulièrement pour objet un système catalytique constitué d'hétéropolyanion auquel sont liés en proportion variable des cations tels que des protons $H^+$, le cation vanadyle $VO^{2+}$ ou des cations métalliques comme le cuivre : $Cu^{2+}$, le Fer : $Fe^{3+}$ ou le Cobalt $Co^{2+}$. Le système catalytique objet de l'invention convient particulièrement pour la réaction d'oxydéshydrogénation d'acides ou d'esters carboxyliques saturés en acides ou esters insaturés correspondants, en particulier pour la réaction d'oxydéshydrogénation de l'acide isobutyrique en acide méthacrylique ainsi que pour des réactions d'oxydation telles que l'oxydation de la méthacroléine en acide méthacrylique.

On connaît, depuis longtemps, des systèmes catalytiques qui permettent la réalisation de l'oxydéshydrogénation d'acides ou d'esters carboxyliques saturés en acides insaturés ou esters correspondants. C'est ainsi qu'il est décrit dans le brevet français FR-A-2 497 795 un catalyseur de formule générale : $FeP_xMe_yO_z$ dans laquelle Me est Li, Na, K, Rb, Cs, Mg, Sr, ou Ba : un tel catalyseur convient pour la fabrication de l'acide méthacrylique à partir de l'acide isobutyrique. On a aussi décrit (J. catal 98, 401, 1986), la synthèse de l'acide méthacrylique à partir de l'acide isobutyrique en présence d'un mélange binaire d'oxydes constitué d'oxyde de vanadium ($V_2O_5$) et d'anhydride phosphorique ($P_2O_5$). Dans cette même publication des tests comparatifs ont été aussi réalisés à partir de catalyseurs de formule $H_5PMo_{10}V_2O_{40}$. Les résultats indiqués montrent que la sélectivité en acide méthacrylique plafonne autour de 60% quel que soit le catalyseur mis en oeuvre.

Des systèmes catalytiques à base d'hétéropolyanions ont également été décrits dans d'autres publications comme catalyseurs de la réaction de déshydrogénation oxydante de l'acide isobutyrique, ou de ses esters, en acide méthacrylique ou en ses esters.

C'est ainsi que la demande allemande DE-A-27 22 375, décrit des catalyseurs obtenus par concentration d'une solution aqueuse d'un hétéropolyacide de composition $H_5PMo_{10}V_2O_{40}$ et d'un sel de cuivre, éventuellement en présence d'un sel alcalin : le résidu sec étant ensuite calciné. De tels catalyseurs permettent d'atteindre des sélectivités en acide méthacrylique de 71-72% et par incorporation de lithium on peut atteindre 74,8%.

Le brevet américain US-A-4 307 247, propose pour la même réaction de déshydrogénation oxydante des catalyseurs à base de molybdène, de vanadium, de cuivre, de phosphore, de bismuth auquel on ajoute un métal alcalin du groupe du potassium, du rubidium ou du césium et éventuellement d'autres métaux, ainsi que de l'oxygène en rapports quantitatifs déterminés.

Le brevet américain US-A-4 314 075, propose pour la même réaction des catalyseurs semblables à base des éléments suivants : molybdène, vanadium, cuivre et phosphore et d'un métal alcalin choisi parmi le potassium, le rubidium ou le césium et un ou plusieurs autres métaux ainsi que l'oxygène dans des rapports quantitatifs déterminés. Tous les métaux sont présents au cours de la réaction hydrothermale qui conduit à la solution aqueuse d'un hétéropolyanion. Les sélectivités en acide méthacrylique atteintes en présence de ces catalyseurs se situent entre 60 et 70%.

Il est décrit également dans le brevet américain US-A-4 522 934 des catalyseurs pour cette réaction d'oxydéshydrogénation de formule générale $H_{3+x}PV_xW_{3-x}Mo_9O_{40}$ avec $0<x<3$. Le système $H_{3,5}PMo_9V_{0,5}W_{2,5}O_{40}$ supporté sur silice permet d'atteindre une conversion de l'acide isobutyrique de 93% et une sélectivité en acide méthacrylique de 79%.

Tous ces systèmes catalytiques dérivés des hétéropolyanions présentent toutefois le défaut moyen d'avoir une stabilité limitée dans le temps dans les conditions de réaction d'oxydéshydrogénation.

Dans le brevet européen EP-A-0255 639 il est décrit des catalyseurs pour l'oxydéshydrogénation de l'acide isobutyrique en acide méthacrylique à base d'hétéropolyacides comprenant du molybdène, du vanadium et du phosphore ou de leurs sels métalliques. Les sélectivités en acide méthacrylique atteintes en présence de ces catalyseurs se situent entre 50 et 76% et les conversions se situent entre 47 et 90,6%.

Le brevet européen EP-A-0 284 947 propose également des catalyseurs pour l'oxydéshydrogénation de l'acide isobutyrique à base d'hétéropolyacides comprenant du molybdène, du vanadium et du phosphore ou de leurs dérivés métalliques, mais les sélectivités ne sont qu'entre environ 63 et 75%.

Il est aussi dans l'art antérieur que ce type de catalyseurs peut être utilisé pour réaliser l'oxygénation de la méthacroléine en acide méthacrylique avec de bons rendements. Proc. 7th Intern. Congress Catalysis, Tokyo (1980), p. 755.

Le brevet français FR-A- 2 407 022 propose un catalyseur à base d'anion phosphovanadomolybdique, de cuivre et d'un ou plusieurs éléments métalliques pour l'oxydation de méthacroleine en acide méthacrylique.

Le besoin se fait donc sentir de disposer de catalyseurs du type hétéropolyanions permettant d'obtenir des sélectivités en acides carboxyliques insaturés acceptables industriellement associées à des conversions élevées des acides carboxyliques saturés de départ soumis à la réaction catalytique d'oxydéshydrogénation, ces catalyseurs doivent aussi présenter de bons taux de conversion des aldéhydes dans les réactions d'oxy-

2

dation des aldéhydes en acides, en particulier, lors de l'oxydation de la méthacroléine en acide méthacrylique. Par ailleurs il est aussi nécessaire que ces catalyseurs restent stables dans le temps dans les conditions réactionnelles.

La présente invention a pour objet un système catalytique à base d'oxygène, de vanadium, de phosphore et de molybdène caractérisé en ce que le système catalytique répond à la formule générale : $H_mX_nMe_pPMo_{12-x}V_xO_{40}$ dans laquelle X représente le cation $VO^{2+}$ avec $0<n\leqq2$

H représente un proton avec $0\leqq m<4$ ; $0\leqq x\leqq3$ ; Me est un ion métallique en particulier un ion d'un métal de transition avec $0\leqq p<t$, t dépendant de la charge de l'ion correspondant. Par exemple, t est égal à 2 pour le cuivre et le cobalt, et est égal à 1,333 pour le fer.

En particulier, m+2n+qp = 4, q étant la charge de l'ion Me.

Selon l'invention les systèmes catalytiques ont la formule $H_m X_n Me_p PMo_{12-x} V_xO_{40}$ dans laquelle Me est un ion métallique en particulier un ion d'un métal de transition tel que le cuivre, le cobalt, le fer, l'argent, le nickel, le manganèse ou la magnésium. Me peut également représenter un métal alcalin.

L'invention porte également sur un procédé de fabrication du système catalytique qui vient d'être défini, suivant lequel on prépare de façon connue en soi l'hétéropolyacide $H_4PMo_{12-x}V_xO_{40}$, puis on introduit le cation $VO^{2+}$ et le cas échéant au moins un cation Me, notamment par réaction avec de l'hydroxyde de baryum et du sulfate de vanadyle, et, le cas échéant un ou des sulfates d'un ou de cations Me, avec précipitation du sulfate de baryum, et, le cas échéant, à partir du composé hétéropolyacide obtenu à l'étape précédente, on prépare le système catalytique recherché par substitution d'au moins une partie des protons par réaction avec un carbonate ou hydroxyde du ou des cations correspondants Me à associer.

Les systèmes catalytiques objet de l'invention sont donc avantageusement fabriqués en deux étapes. Dans une première étape on fabrique de façon connue l'hétéropolyacide de formule $H_4PMo_{11}VO_{40}$ (Voir Courtin, Rev de chimie Minérale 8, 75, 1971), ou, de façon plus générale, l'hétéropolyacide de formule $H_4PMo_{12-x}V_xO_{40}$.

## 1) Préparation de l'hétéropolyacide de formule $H_4PMo_{11}VO_{40}$

On mélange en quantité équimoléculaire du métavanadate de sodium (Na $VO_3$) et du monohydrogénophosphate de sodium bihydraté que l'on acidifie avec de l'acide chlorhydrique. On ajoute ensuite du molybdate de sodium ($Na_2MoO_4$), (11 moles pour 1 mole des réactifs précédents), et l'on acidifie le mélange réactionnel avec de l'acide chlorhydrique. La solution est ensuite traitée à l'éther, décantée et cristallisée.

## 2) Introduction des cations ($VO^{2+}$ et, le cas échéant, $Cu^{2+}$, $Fe^{3+}$, $Co^{2+}$ ...)

L'hétéropolyacide est dissous dans l'eau, puis pour 1 mole de cet hétéropolyacide, on ajoute n moles d'hydroxyde de baryum solide ($Ba(OH)_2$, $8H_2O$) et n moles de sulfate de vanadyle ($VOSO_4$, $5H_2O$). On peut remplacer une partie du sulfate de vanadyle par un ou des sulfates du ou des cations Me à introduire, ce en fonction du nombre de protons que l'on veut substituer dans l'hétéropolyacide ainsi que de la nature du cation pour obtenir la formule générale désirée. Le sulfate de baryum formé est ensuite éliminé par filtration, la composition catalytique est extraite par cristallisation ou par évaporation.

A partir des composés $H_{4-2n}(VO)_nPMo_{11}VO_{40}$ où $0<n\leqq2$, il est possible d'obtenir certains composés $H_m(VO)_nMe_pPMo_{11}VO_{40}$ où Me est un cation métallique comme $Cu^{2+}$, $Co^{2+}$ par substitution des protons avec un sel de carbonate ou d'hydroxyde du cation correspondant à associer. On procède alors de la manière suivante : à une partie de $H_{4-2n}(VO)_nPMo_{11}VO_{40}$ dissoute dans de l'eau distillée, on ajoute une partie de carbonate du cation à associer en quantité correspondante à la formule désirée. L'extraction du composé solide est alors réalisée par évaporation, comme précédemment.

La présente invention a également pour objet un procédé d'oxydéshydrogénation d'acides carboxyliques saturés pour former des acides carboxyliques $\alpha,\beta$ insaturés. Ce procédé consiste à mettre en contact les acides carboxyliques saturés avec de l'oxygène moléculaire ou un gaz contenant de l'oxygène et le cas échéant un gaz diluant inerte en phase vapeur, à une température de réaction comprise entre 280°C et 400°C de préférence entre 300 et 340°C en présence d'un système catalytique comprenant un catalyseur de formule générale $H_mX_nMe_pPMo_{12-x} V_xO_{40}$ dans laquelle m, n, p et x ont les valeurs précitées dans un rapport molaire à l'acide saturé ne dépassant pas 2. De préférence cette réaction est réalisée en présence de vapeur d'eau ce qui permet un bon déroulement de la réaction.

Ce type de réaction se déroule généralement dans un réacteur tubulaire à lit fixe dont le fonctionnement général peut être décrit par le modèle théorique du réacteur piston. Les conditions de marche dans un tel circuit sont généralement les suivantes : une charge envoyée sur le catalyseur, préalablement mis en forme (pastillé, extrudé) ou déposé sur un support adéquat, comprend un mélange gazeux généralement préchauffé constitué d'acide saturé, d'oxygène moléculaire, d'eau et d'un diluant gazeux inerte (tel que l'azote ou l'hélium). La proportion d'acide dans ce mélange est généralement comprise entre 1 et 8% exprimée en moles, la proportion d'oxygène moléculaire est, en mole, 0,2 à 5 fois supérieure à celle de l'acide saturé, de préférence de 0,5 à 2. L'eau introduite dans le réacteur, avec un rapport molaire compris entre 0 et 15 par rapport à l'acide saturé s'entend, bien sûr, à l'exclusion de la quantité d'eau générée, comme il est bien connu, par la réaction de déshydrogénation oxydante proprement dite. Le temps de contact entre la charge et le catalyseur est compris entre 0,1 et 50 secondes et de préférence entre 0,2 et 4 secondes.

Un autre objet de la présente invention consiste dans l'utilisation des catalyseurs dans la réaction d'oxydation de la méthacroléine en acide méthacrylique. Cette réaction est réalisée de façon classique dans les mêmes conditions opératoires que la réaction d'oxydéshydrogénation des acides carboxyliques saturés.

Les exemples suivants illustrent la présente invention.

## EXEMPLE 1

Catalyseur mis en oeuvre $VOH_2PMO_{11}VO_{40}$

Une solution contenant 0,1 mole de métavanadate de sodium $NaVO_3$ dans 0,5 dm³ d'eau, 0,1 mole de monohydrogénophosphate de sodium bihydraté (17,8 g) est acidifiée par 0,12 mole d'acide chlorhydrique (10 ml HCl 35%). A cette solution il est ajouté goutte à goutte une solution constituée de 1,1 mole de molybdate de sodium bihydraté (266 g) dilué dans 0,5 dm³ d'eau. La solution ainsi obtenue est acidifiée par 4,5 moles d'acide chlorhydrique (400 ml HCl 35%). Après refroidissement de cette solution 400 ml d'éther di-éthylique sont ajoutés. Après décantation (trois phases), une couche lourde qui contient le composé désiré est recueillie (200 ml), dans laquelle 100 ml d'eau sont ajoutés. L'éther est éliminé de la solution par agitation à l'air. Le composé est extrait de la solution finale par cristallisation à 4°C. Les cristaux ainsi obtenus de formule $H_4PMo_{11}VO_{40}$, $29H_2O$, s'effleurissent rapidement à l'air pour conduire à $H_4PMo_{11}VO_{40}$, $13H_2O$. Le rendement en hétéropolyacide est de 97% par rapport aux composés mis en oeuvre.

On dissout 10 g de $H_4PMo_{11}VO_{40}$ ($4,95 \cdot 10^{-3}$ mole) dans 10 ml d'eau distillée, 1,56 g de $Ba(OH)_2$, $8 H_2O$ ($4,95 \cdot 10^{-3}$ mole) est ajouté lentement par petites fractions à la solution sous agitation, puis on introduit 1,25 g de $VOSO_4$, $5H_2O$.($4,95 \cdot 10^{-3}$ mole). Un précipité de sulfate de baryum apparaît, il est éliminé par filtration. Ce précipité est lavé plusieurs fois par des petites fractions d'eau distillée. Les eaux de lavages sont recueillies et ajoutées à la solution mère. 10 g de $VOH_2PMo_{11}VO_{40}$ sont extraits de la solution par évaporation de l'eau par barbotage à l'azote pendant trois heures à température ambiante, ou par évaporation sous vide avec un chauffage modéré à 60°C sur un évaporateur rotatif.

0,66 gramme du catalyseur ainsi obtenu est introduit dans un réacteur tubulaire à lit fixe. Un prétraitement thermique de ce catalyseur est réalisé sous débit d'air ; avec une vitesse de chauffe de 225°C/h pendant une heure pour atteindre 250°C et de maintenir à cette température pendant 2 heures avant de lui faire supporter une augmentation de température de 250°C à 320°C en 1 heure.

Dans le même réacteur on introduit le mélange réactionnel suivant :

2,2% en moles d'acide isobutyrique

5,2% en moles d'oxygène moléculaire

3,8% en moles de vapeur d'eau

88,8% en moles d'azote diluant

Le débit du mélange gazeux est de 165 ml/minute ce qui représente un temps de passage du gaz par rapport au volume du catalyseur introduit de 0,2 seconde, la température dans le réacteur est de 320°C. Les résultats obtenus sont reportés dans le Tableau 1.

## EXEMPLE 2

Catalyseur utilisé : $(VO)_{0,5}H_3PMo_{11}VO_{40}$

On dissout 10 g de $H_4PMo_{11}VO_{40}$ ($4,95 \cdot 10^{-3}$ mole), obtenu selon la méthode décrite précédemment dans 10 ml d'eau distillée. 0,78 gramme de $Ba(OH)_2$, $8H_2O$ ($2,5 \cdot 10^{-3}$ mole) est ajouté lentement par petites fractions à la solution sous agitation puis on ajoute 0,625 g de $VOSO_4$, $5H_2O$ ($2,5 \cdot 10^{-3}$ mole). Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de $(VO)_{0,5}H_3PMo_{11}VO_{40}$ sont extraits par évaporation comme dans l'Exemple 1.

Les performances catalytiques de ce système, pour l'oxydéshydrogénation de l'acide isobutyrique, sont évaluées de manière identique à celle décrite dans l'Exemple 1. Les résultats sont regroupés dans le Tableau 2.

## EXEMPLE 3

Catalyseur utilisé : $(VO)_2PMo_{11}VO_{40}$

On dissout 10 g de $H_4PMo_{11}VO_{40}$ (4,95 $10^{-3}$ mole) dans 10 ml d'eau distillée, puis on ajoute 3,12 g de $Ba(OH)_2$, $8H_2O$ (9,9 $10^{-3}$ mole) lentement par petites fractions à la solution sous agitation puis 2,5 g de $VOSO_4$, $5H_2O$ (9,9 $10^{-3}$ mole). Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de $(VO)_2PMo_{11}VO_{40}$ sont extraits par évaporation comme dans l'Exemple 1.

Les performances catalytiques de ce système, pour l'oxydéshydrogénation de l'acide isobutyrique, sont évaluées de manière identique à celle décrite dans l'Exemple 1. Les résultats sont regroupés dans le Tableau 3.

## EXEMPLE 4

Catalyseur mis en oeuvre : $(VO)_{1,5}Cu_{0,5}PMo_{11}VO_{40}$

On dissout 10 g de $H_4PMo_{11}VO_{40}$ (4,95 $10^{-3}$ mole), obtenu selon la méthode décrite précédemment dans 10 ml d'eau distillée, on ajoute 3,12 g de $Ba(OH)_2$, $8H_2O$ (9,9 $10^{-3}$ mole) lentement par petites fractions à la solution sous agitation puis 1,875 g de $VOSO_4$, $5H_2O$ (7,5 $10^{-3}$ mole) et 0,624 g de $CuSO_4$, $5H_2O$ (2,5 $10^{-3}$ mole) sont introduits. Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de $(VO)_{1,5}Cu_{0,5}PMo_{11}VO_{40}$ sont extraits par évaporation comme dans l'Exemple 1.

Les performances catalytiques de ce système, pour l'oxydéshydrogénation de l'acide isobutyrique, sont évaluées de manière identique à celle décrite dans l'Exemple 1 avec une composition du gaz réactionnel suivante :

2,2% en moles d'acide isobutyrique
2,8% en moles d'oxygène moléculaire
3,8% en moles de vapeur d'eau
91,2% en moles d'azote diluant
Les résultats sont regroupés dans le Tableau 4.

## EXEMPLE 5

Catalyseur utilisé : $VOHCu_{0,5}PMo_{11}VO_{40}$

On dissout 10 g de $H_4PMo_{11}VO_{40}$ (4,95 $10^{-3}$ mole), dans 10 ml d'eau distillée, et on ajoute 1,56 g de $Ba(OH)_2$, $8H_2O$ (4,95 $10^{-3}$ mole) lentement par petites fractions à la solution sous agitation puis 1,25 g de $VOSO_4$, $5H_2O$ (4,95 $10^{-3}$ mole). Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de $VOH_2PMo_{11}VO_{40}$ sont extraits de la solution par évaporation de l'eau par barbotage à l'azote pendant 3 heures à température ambiante, ou par évaporation sous vide avec un chauffage modéré à 60°C dans un évaporateur rotatif.

A 10 g (4,2 $10^{-3}$ mole) de ce système dissous dans 10 ml d'eau distillée on ajoute 0,232 g de $CuCO_3$,$Cu(OH)_2$ (1,05 $10^{-3}$ mole). 10 g de $VOHCu_{0,5}PMo_{11}VO_{40}$ sont extraits par évaporation comme dans l'Exemple 1.

Les performances catalytiques de ce système, pour l'oxydéshydrogénation de l'acide isobutyrique, sont évaluées de manière identique à celle décrite dans l'Exemple 1. Les résultats sont regroupés dans le Tableau 5.

## EXEMPLE 6

Catalyseur utilisé : $VOHCo_{0,5}PMo_{11}VO_{40}$

On dissout 10 g de $H_4PMo_{11}VO_{40}$ (4,95 $10^{-3}$ mole), dans 10 ml d'eau distillée, et on ajoute 1,56 g de Ba

(OH)$_2$, 8 H$_2$O (4,95 10$^{-3}$ mole) lentement par petites fractions à la solution sous agitation puis 1,25 g de VOSO$_4$, 5H$_2$O (4,95 10$^{-3}$ mole). Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de VOH$_2$PMo$_{11}$VO$_{40}$ sont extraits de la solution par évaporation de l'eau par barbotage à l'azote pendant 3 heures à température ambiante, ou par évaporation sous vide avec un chauffage modéré à 60°C dans un évaporateur rotatif.

On ajoute à 10 g (4,2 10$^{-3}$ mole) de ce système dissous dans 10 ml d'eau distillée 0, 2 50 g de CoCO$_3$, (2,1 10$^{-3}$ mole) sous forme de la quantité correspondante de carbonate de cobalt hydraté. 10 g de VOHCo$_{0,5}$PMo$_{11}$VO$_{40}$ sont extraits par évaporation comme dans l'Exemple 1.

Les performances catalytiques de ce système, pour l'oxydéshydrogénation de l'acide isobutyrique, sont évaluées de manière identique à celle décrite dans l'Exemple 1. Les résultats sont regroupés dans le Tableau 6.

EXEMPLE 7

Catalyseur utilisé : VOHFe$_{0,33}$PMo$_{11}$VO$_{40}$

On dissout 10 g de H$_4$PMo$_{11}$VO$_{40}$ (4,95 10$^{-3}$ mole), dans 10 ml d'eau distillée, et on ajoute 1,56 g de Ba (OH)$_2$, 8 H$_2$O (4,95 10$^{-3}$ mole) lentement par petites fractions à la solution sous agitation puis 1,25 g de VOSO$_4$, 5H$_2$O (4,95 10$^{-3}$ mole). Un précipité de sulfate de baryum apparaît qui est éliminé par filtration. Ce précipité est lavé plusieurs fois par des petites fractions d'eau distillée. Les eaux de lavages sont recueillies et ajoutées à la solution mère filtrée. 10 g de VOH$_2$PMo$_{11}$VO$_{40}$ sont extraits de la solution par évaporation de l'eau par barbotage à l'azote pendant 3 heures à température ambiante, ou par évaporation sous vide avec un chauffage modéré à 60°C dans un évaporateur rotatif.

10 g de ce système (4,2 10$^{-3}$ mole) dissous dans 10 ml d'eau distillée sont agités avec de l'hydroxyde ferrique (1,4 10$^{-3}$ mole) fraîchement précipité. Cet hydroxyde ferrique est obtenu à partir de 0,57 g de nitrate de fer III à 9 molécules d'eau, mis en solution, précipité en milieu ammoniacal, filtré et lavé.

Les performances catalytiques de ce système, pour l'oxydéshydrogénation de l'acide isobutyrique, sont évaluées de manière identique à celle décrite dans l'Exemple 1. Les résultats sont regroupés dans le Tableau 7.

EXEMPLE 8

Le système VOH$_2$PMo$_{11}$VO$_{40}$ est calciné à 120°C pendant 3 heures puis pastillé. 25 grammes des pastilles annulaires ainsi obtenues, diluées dans du carbure de silicium sont introduites dans un réacteur chauffé par un bain de sel à 200°C. La température du bain de sel est ensuite augmentée de 250 à 300°C en une heure puis on introduit dans le réacteur un mélange réactionnel liquide et gazeux, après passage sur un vaporiseur à 200°C.

Le mélange réactionnel est constitué :
2,2% en moles d'acide isobutyrique
5,2% en moles d'oxygène moléculaire
3,8% en moles de vapeur d'eau
88,8% en moles d'azote diluant

Les performances catalytiques de ce système, pour l'oxydéshydrogénation de l'acide isobutyrique, sont évaluées de manière identique à celle décrite dans l'Exemple 1. Les résultats sont regroupés dans le Tableau 8.

EXEMPLE 9

Oxydation de la méthacroléine en acide méthacrylique : Catalyseur mis en oeuvre : VOHCu$_{0,5}$PMo$_{11}$VO$_{40}$

On met en oeuvre dans un réacteur un mélange réactionnel constitué en moles de :
3% de méthacroléine
13,4% d'air
53,6% d'azote
30% d'eau
La réaction est conduite à pression atmosphérique à 310°C, le temps de passage du gaz par rapport au volume du catalyseur est de 2,4 secondes.

La conversion de la méthacroléine est de 90%, la sélectivité de l'acide méthacrylique est de 75%.

TABLEAU 1

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0 | 94,5 | 75,7 | 6,4 | 16,4 | 1,4 | 0 |
| 2 | 93,1 | 77,7 | 5,5 | 15,6 | 1,1 | 0 |
| 4 | 92,5 | 77,9 | 5,4 | 15,7 | 1 | 0 |
| 6 | 92,2 | 77,9 | 5,3 | 15,8 | 0,8 | 0 |
| 10 | 91,6 | 77,6 | 5,4 | 16,2 | 0,8 | 0 |
| 14 | 91,2 | 77,5 | 5,4 | 16,3 | 0,8 | 0 |
| 16 | 91,2 | 77,5 | 5,4 | 16,3 | 0,8 | 0 |
| 18 | 90,9 | 77,6 | 5,3 | 16,2 | 0,8 | 0 |
| 23 | 90,4 | 77,1 | 5,5 | 16,6 | 0,7 | 0 |
| 63 | 89,0 | 76,6 | 6,0 | 16,8 | 0,5 | 0 |

TABLEAU 2

| TEMPS heures | CONVER-SION % | SELECTI-VITE AMA % % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0 | 91,5 | 72,3 | 10,5 | 16,2 | 0,9 | 0 |
| 2 | 89,2 | 72,2 | 10,4 | 16,7 | 0,6 | 0 |
| 4 | 89,4 | 73,3 | 10,1 | 16,1 | 0,5 | 0 |
| 6 | 90,9 | 72,9 | 10,1 | 16,4 | 0,8 | 0 |
| 8 | 90,5 | 73 | 10,1 | 16,4 | 0,4 | 0 |
| 10 | 93,3 | 73 | 9,5 | 16,9 | 0,5 | 0 |
| 12 | 90 | 72,7 | 8,7 | 17,7 | 0,8 | 0 |

TABLEAU 3

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0 | 81,9 | 70,6 | 6,4 | 21, | 1,3 | 0 |
| 3 | 86,3 | 72,6 | 5,9 | 20,2 | 1,1 | 0 |
| 4 | 86 | 72,5 | 6,1 | 20,3 | 1,1 | 0 |
| 5 | 82 | 72,7 | 6,1 | 20,2 | 1 | 0 |
| 7 | 81 | 73,3 | 6,1 | 19,8 | 0,9 | 0 |
| 9 | 78,6 | 72,6 | 5,9 | 19,8 | 0,8 | 0 |
| 11 | 79,3 | 72,7 | 6,4 | 20,0 | 0,8 | 0 |
| 13 | 73,6 | 72,4 | 6,4 | 20,3 | 0,8 | 0 |
| 15 | 76,4 | 72,5 | 6,4 | 20,2 | 0,8 | 0 |
| 17 | 72,6 | 72,4 | 6,5 | 20,1 | 0,8 | 0 |

TABLEAU 4

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0 | 93,1 | 77,15 | 8,2 | 12,2 | 1,1 | 0,8 |
| 1 | 93,7 | 79,6 | 7,8 | 10,6 | 1 | 0,5 |
| 2 | 93,7 | 79,7 | 7,8 | 10,4 | 1 | 0,6 |
| 3 | 93,5 | 79,4 | 7,8 | 10,5 | 1 | 0,7 |
| 5 | 93,4 | 79,2 | 7,8 | 10,5 | 1 | 1 |
| 6 | 93,2 | 78,6 | 8,3 | 11,9 | 0,6 | 0,8 |
| 8 | 93,3 | 79,4 | 7,8 | 10,3 | 1 | 1 |
| 25 | 92,7 | 79 | 8 | 10,4 | 0,9 | 1,1 |

TABLEAU 5

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0,5 | 91,9 | 77,6 | 7,4 | 12,6 | 2,6 | 0 |
| 1 | 91,9 | 76,4 | 6,7 | 12 | 2,2 | 2,6 |
| 3 | 91,2 | 76,9 | 6,6 | 12,2 | 6,6 | 2,3 |
| 5 | 90,9 | 77,3 | 6,3 | 12,4 | 6,3 | 2,1 |
| 7 | 91,2 | 77,6 | 6,5 | 12,1 | 1,8 | 2 |
| 9 | 91,7 | 77,8 | 6,2 | 12,4 | 1,7 | 1,9 |
| 11 | 91,4 | 77,9 | 6,3 | 12,4 | 1,7 | 1,7 |

TABLEAU 6

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 2 | 81 | 75,5 | 7,8 | 14,8 | 0,7 | 1,1 |
| 3 | 81,2 | 75,8 | 7,8 | 14,7 | 0,7 | 1 |
| 5 | 79,6 | 74,6 | 8,4 | 15,5 | 0,7 | 0,9 |
| 7 | 78,4 | 74,5 | 8,4 | 15,6 | 0,7 | 0,8 |
| 10 | 79,3 | 75 | 8,4 | 15,4 | 0,6 | 0,6 |
| 12 | 78,3 | 74,3 | 8,6 | 15,9 | 0,6 | 0,6 |
| 15 | 79,4 | 74,5 | 8,7 | 15,7 | 0,6 | 0,5 |

TABLEAU 7

| TEMPS heures | CONVER-SION % | SELECTI-VITE AMA % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0 | 85,2 | 76,3 | 6,9 | 14,2 | 1,5 | 1,1 |
| 1 | 84,9 | 76,4 | 6,6 | 14 | 1,2 | 1,7 |
| 3,5 | 85 | 76,5 | 6,8 | 14,7 | 1 | 1,1 |
| 6 | 80,6 | 76,9 | 6,4 | 15,6 | 0,8 | 0,3 |
| 8,5 | 86,6 | 76,2 | 6,9 | 15 | 0,9 | 1 |
| 11 | 80,1 | 75,5 | 6,9 | 15,1 | 0,8 | 1,8 |
| 14,5 | 77,6 | 76,6 | 6,3 | 16,3 | 0,7 | 0 |
| 17 | 83,7 | 76,3 | 7,7 | 16,6 | 0,8 | 0,5 |
| 19,5 | 80,4 | 77,0 | 7 | 15,3 | 0,8 | 0 |
| 22 | 80,2 | 76,1 | 6,9 | 15,5 | 0,8 | 0,8 |
| 24,5 | 80,2 | 76,1 | 7,1 | 14,9 | 0,8 | 1 |

TABLEAU 8

| TEMPS heures | CONVER-SION % | SELECTI-VITE AMA % % | SELECTI-VITE PROPENE % | SELECTI-VITE ACETONE % | SELECTI-VITE Acide Acétique % | SELECTI-VITE CO/CO2 % |
|---|---|---|---|---|---|---|
| 0,2 | 96,1 | 71,3 | 8 | 16,9 | 1,7 | 2,5 |
| 1 | 92,6 | 72 | 5,8 | 17,7 | 1,1 | 1,4 |
| 4 | 90,8 | 71,6 | 7,3 | 18,6 | 0,7 | 1,1 |
| 5 | 90,9 | 71,5 | 7,5 | 18,4 | 0,8 | 1 |
| 11 | 90,2 | 69,9 | 8,6 | 18,3 | 1 | 1,3 |
| 18 | 90 | 69,7 | 9 | 18,2 | 1,1 | 0,9 |
| 26 | 88,5 | 69,4 | 9 | 18,3 | 0,6 | 1 |
| 27 | 88,4 | 70,2 | 8,1 | 18,1 | 0,7 | 1,1 |

**Revendications**

1.  Système catalytique à base d'oxygène, de vanadium, de phosphore et de molybdène, caractérisé en ce que le système catalytique répond à la formule générale :

$$H_mX_nMe_pPMo_{12-x}V_xO_{40}$$

dans laquelle X représente le cation $vo^{2+}$ avec $0<n\leqq2$

H représente des protons avec $0\leqq m<4$ ; $0\leqq x\leqq3$ ;

Me est un ion métallique en particulier un ion d'un métal de transition avec $0\leqq p<t$, t dépendant de la charge de l'ion correspondant.

2.  Système catalytique selon la revendication 1, caractérisé en ce que Me est un ion d'un métal de transition.

3.  Système catalytique selon la revendication 1, caractérisé en ce que le métal est choisi parmi le cuivre, le cobalt, le fer, l'argent, le nickel, le manganèse ou le magnésium.

4.  Procédé de fabrication du système catalytique tel que défini à l'une des revendications 1 à 3, caractérisé par le fait qu'on prépare de façon connue en soi l'hétéropolyacide $H_4PMo_{12-x}V_xO_{40}$, puis qu'on introduit le cation $VO^{2+}$, et, le cas échéant, au moins un cation Me, notamment par réaction avec de l'hydroxyde de baryum et du sulfate de vanadyle, et, le cas échéant, un ou des sulfates d'un ou de cations Me, avec précipitation du sulfate de baryum, et que, le cas échéant, à partir du composé hétéropolyacide obtenu à l'étape précédente, on prépare le système catalytique recherché par substitution d'au moins une partie des protons par réaction avec un carbonate ou hydroxyde du ou des cations correspondants Me à associer.

5.  Application du système catalytique selon l'une quelconque des revendications 1 à 3 à la réaction d'oxydéshydrogénation d'acides carboxyliques saturés.

6.  Application du système catalytique selon l'une quelconque des revendications 1 à 3 à la réaction d'oxydation d'aldéhydes en acides.

**Patentansprüche**

1.  Katalytisches System auf Basis von Sauerstoff, Vanadium, Phosphor und Molybdän, dadurch gekennzeichnet, daß das katalytische System der allgemeinen Formel

$$H_mX_nMe_pPMo_{12-x}V_xO_{40}$$

entspricht, in der

X ein $VO^{2+}$-Kation mit $0 < n \leqq 2$ ist,

H für Protonen mit $0 \leqq m < 4$; $0 \leqq x \leqq 3$ steht,

Me ein Metallion ist, vorzugsweise ein Übergangsmetallion mit $0 \leqq p < t$, wobei t abhängig von der Ladung des korrespondierenden Ions ist.

2.  Katalytisches System nach Anspruch 1, dadurch gekennzeichnet, daß Me ein Übergangsmetallion ist.

3.  Katalytisches System nach Anspruch 1, dadurch gekennzeichnet, daß das Metall aus der Gruppe von Kupfer, Cobalt, Eisen, Silber, Nickel, Mangan oder Magnesium ausgewählt ist.

4.  Verfahren zur Herstellung eines katalytischen Systems, wie nach einem der Ansprüche 1 bis 3 beschrieben, dadurch gekennzeichnet, daß man, in an sich bekannter Weise, die Heteropolysäure $H_4PMo_{12-x}V_xO_{40}$ herstellt, dann das $VO^{2+}$-Kation und gegebenenfalls mindestens ein Me-Kation hinzugibt, vorzugsweise durch Reaktion von Bariumhydroxid, Vanadylsulfat und gegebenenfalls einem oder mehreren Sulfat/Sulfaten eines oder mehrerer Me-Kationen mit Fällung von Bariumsulfat, und daß man gegebenenfalls, ausgehend von der in der vorhergehenden Stufe erhaltenen Heteropolysäure, das gesuchte katalytische System durch Substitution mindestens eines Teils der Protonen durch Reaktion mit den Carbonaten oder Hydroxiden des oder der korrespondierenden, zu verbindenden Me-Kationen herstellt.

5.  Verwendung des katalytischen Systems nach einem der Ansprüche 1 bis 3 zur Oxydehydrierung gesättigter Carbonsäuren.

**6.** Verwendung des katalytischen Systems nach einem der Ansprüche 1 bis 3 zur Oxidation von Aldehyden zu Säuren.

## Claims

1. Catalytic system based on oxygen, vanadium, phosphorus and molybdenum, characterised in that the catalytic system corresponds to the general formula:

$$H_mX_nMe_pPMo_{12-x}V_xO_{40}$$

in which X represents the $VO^{2+}$ cation with $0 < n \leqq 2$,

H represents protons with $0 \leqq m < 4$, $0 \leqq x \leqq 3$,

Me is a metal ion, in particular an ion of a transition metal, with $0 \leqq p < t$, t depending on the charge of the corresponding ion.

2. Catalytic system according to Claim 1, characterised in that Me is an ion of a transition metal.

3. Catalytic system according to Claim 1, characterised in that the transition metal is chosen from copper, cobalt, iron, silver, nickel, manganese or magnesium.

4. Process for manufacturing the catalytic system such as defined in one of Claims 1 to 3, characterised in that the heteropolyacid $H_4PMo_{12-x}V_xO_{40}$ is prepared in a manner known per se, in that the $VO^{2+}$ cation and, if appropriate, at least one Me cation are then introduced, especially by reaction with barium hydroxide and vanadyl sulphate and, if appropriate, one or more sulphates of one or more Me cations are introduced, with precipitation of barium sulphate and in that, if appropriate, the sought-after catalytic system is prepared from the heteropolyacid compound obtained in the preceding stage, by substitution of at least a part of the protons by reaction with a carbonate or hydroxide of the corresponding Me cation(s) to be combined.

5. Application of the catalytic system according to any one of Claims 1 to 3 to the oxydehydrogenation reaction of saturated carboxylic acids.

6. Application of the catalytic system according to any one of Claims 1 to 3 to the oxidation reaction of aldehydes to acids.